(19)

Europäisches
Patentamt
European
Patent Office
Office européen
des brevets

(11) **EP 4 517 298 A1**

(12) **EUROPEAN PATENT APPLICATION**
published in accordance with Art. 153(4) EPC

(43) Date of publication:
05.03.2025 Bulletin 2025/10

(21) Application number: 23796263.4

(22) Date of filing: 21.04.2023

(51) International Patent Classification (IPC):
*G01N 21/17* (2006.01)  *G01N 21/49* (2006.01)

(52) Cooperative Patent Classification (CPC):
G01N 21/17; G01N 21/49

(86) International application number:
PCT/JP2023/015877

(87) International publication number:
WO 2023/210521 (02.11.2023 Gazette 2023/44)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 27.04.2022 JP 2022073333

(71) Applicant: National Institute of Advanced
Industrial
Science and Technology
Chiyoda-ku
Tokyo 100-8921 (JP)

(72) Inventors:
• YAMADA Yukio
Tsukuba-shi, Ibaraki 305-8560 (JP)
• YAMADA Toru
Tsukuba-shi, Ibaraki 305-8560 (JP)
• KAWAGUCHI Hiroshi
Tsukuba-shi, Ibaraki 305-8560 (JP)

(74) Representative: J A Kemp LLP
80 Turnmill Street
London EC1M 5QU (GB)

(54) **OPTICAL CHARACTERISTIC VALUE MEASUREMENT DEVICE AND OPTICAL CHARACTERISTIC VALUE MEASUREMENT METHOD**

(57) It is an object to provide an optical characteristic value measuring device and an optical characteristic value measuring method that allow easily obtaining values of a scattering coefficient and an absorption coefficient as optical characteristic values of a scattering/absorbing body.

To achieve the object, provided is an optical characteristic value measuring device 1 provided with a detection unit 2 that includes a light emission unit configured to emit a light from a surface to an inside of an object, a measurement unit configured to measure an intensity of the light reflected at the inside and emitted to an outside of the object at at least two points on the surface mutually different in distance from the light emission unit, and a base plate that is placed to cover the surface between the light emission unit and the measurement unit and increases a reflectance of the light emitted from the inside to the outside, and a computing unit 5 configured to calculate an absorption coefficient and a scattering coefficient of the object based on the intensity obtained by the measurement.

Fig. 2

**Description**

Technical Field

The present invention relates to a technique for measuring an optical characteristic value of a substance.

Background Art

Various techniques for measuring internal information of a scattering/absorbing body, such as a living body, have been designed so far. For example, Patent Literature 1 discloses a technique in which a light is to be incident on a scattering/absorbing body, detected signals are obtained at three or more different incidence-detection distances, and arithmetic processing is performed on the detected signals based on three or more simultaneous relations to the three or more detected signals, thereby extracting internal information of the scattering/absorbing body.

Citation List

Patent Literature

Patent Literature 1: JP H07-49304 A

Summary of Invention

Technical Problem

However, Patent Literature 1 does not mention a reflection (internal reflection) at a surface when a light propagating inside the scattering/absorbing body reaches the surface of the scattering/absorbing body or an absorption characteristic. In view of this, there is a problem that it is not allowed to separately obtain a scattering coefficient and an absorption coefficient of a scattering/absorbing body with the technique disclosed in this patent literature.

The present invention is made to solve the problem as described above. It is an object of the present invention to provide an optical characteristic value measuring device and an optical characteristic value measuring method that allow easily obtaining values of a scattering coefficient and an absorption coefficient as optical characteristic values of a scattering/absorbing body.

Solution to Problem

To solve the above-described problem, the present invention provides an optical characteristic value measuring device comprising: a light emission means configured to emit a light from a surface to an inside of an object; a measurement means configured to measure an intensity of the light reflected at the inside and emitted to an outside of the object at at least two points on the surface mutually different in distance from the light emission means; a light reflection means that is placed to cover the surface between the light emission means and the measurement means and is configured to increase a reflectance of the light emitted from the inside to the outside; and a computing means configured to calculate an absorption coefficient and a scattering coefficient of the object based on the intensity obtained by the measurement.

To solve the above-described problem, the present invention provides an optical characteristic value measuring device, comprising: a measurement means that is disposed on a surface of an object and is configured to measure an intensity of a light emitted from an inside to an outside of the object; a light emission means configured to selectively emit the light from the surface to the inside at at least three points on the surface mutually different in distance from the measurement means; a light reflection means that is placed to cover the surface between the light emission means and the measurement means and is configured to increase a reflectance of the light emitted from the inside to the outside; and a computing means configured to calculate an absorption coefficient and a scattering coefficient of the object based on the intensity obtained by the measurement.

To solve the above-described problem, the present invention provides an optical characteristic value measuring method, in an optical characteristic value measuring device in which a light reflection means is placed between an emission position of a light from a surface to an inside of an object and a measurement position at which an intensity of the light reflected at the inside and emitted to an outside of the object is measured, and the light reflection means is configured to increase a reflectance of the light emitted from the inside to the outside covers the surface, the method comprising: a first step of emitting the light from the surface to the inside; a second step of measuring the intensity of the light reflected at the inside and emitted to the outside of the object at at least two points on the surface mutually different in distance from the emission position; and a third step of calculating an absorption coefficient and a scattering coefficient of the object based on the intensity obtained by the measurement in the second step.

Advantageous Effects of Invention

According to the present invention, it is possible to provide the optical characteristic value measuring device and the optical characteristic value measuring method that allow easily obtaining values of a scattering coefficient and an absorption coefficient as optical characteristic values of a scattering/absorbing body.

Brief Description of Drawings

Fig. 1 is a block diagram illustrating an overall configuration of an optical characteristic value measuring device 1 according to Embodiment 1 of the present invention.
Fig. 2 is a diagram illustrating a structure of an optical probe constituting a detection unit 3 illustrated in Fig. 1. Fig. 2(a) is a bottom view, and Fig. 2(b) is a side view.
Fig. 3 is a flowchart illustrating an operation of the optical characteristic value measuring device illustrated in Fig. 1.
Fig. 4 is a first diagram for describing a principle of a calculation method of an absorption coefficient and a scattering coefficient executed in Step S3 of Fig. 3.
Fig. 5 is a second diagram for describing the principle of the calculation method of the absorption coefficient and the scattering coefficient executed in Step S3 of Fig. 3.
Fig. 6 is a diagram illustrating another example (second example) of the optical probe illustrated in Fig. 2. Fig. 6(a) is a bottom view, and Fig. 6(b) is a side view.
Fig. 7 is a diagram illustrating a third example of the optical probe illustrated in Fig. 2. Fig. 7(a) is a bottom view, and Fig. 7(b) is a side view.
Fig. 8 is a diagram illustrating a fourth example of the optical probe illustrated in Fig. 2. Fig. 8(a) is a bottom view, and Fig. 8(b) is a side view.
Fig. 9 is a diagram illustrating a fifth example of the optical probe illustrated in Fig. 2. Fig. 9(a) is a bottom view, and Fig. 9(b) is a side view.
Fig. 10 is a bottom view illustrating a sixth example of the optical probe illustrated in Fig. 2.
Fig. 11A is a schematic diagram illustrating a state where a voltage is applied for describing a function of a variable reflectance film 20 used in an optical probe constituting a detection unit 2 according to Embodiment 2 of the present invention.
Fig. 11B is a schematic diagram illustrating a state where a voltage is not applied for describing the function of the variable reflectance film 20 used in the optical probe constituting the detection unit 2 according to Embodiment 2 of the present invention.
Fig. 12 is a side view illustrating a structure of the optical probe when a measurement is performed with three different SD distances in Embodiment 2 of the present invention.
Fig. 13 is a side view illustrating a structure of the optical probe when a measurement is performed with two different SD distances in Embodiment 2 of the present invention.
Fig. 14 is a graph for describing a modulation method of an internal reflectance of the variable reflectance film 20 illustrated in Fig. 13. Fig. 14(a) illustrates a temporal change of an internal reflectance $r_d$ of the variable reflectance film 20. Fig. 14(b) illustrates a temporal change of an intensity $I_1$ of a detected light measured by a measurement unit D1 illustrated in Fig. 13.
Fig. 15 is a side view for describing an optical piping phenomenon in which a light transmits through a gap 18.
Fig. 16 is a side view for describing an optical piping phenomenon in which a light transmits through a transparent coating layer 17.
Fig. 17A is a side view illustrating a structure of an optical probe according to Embodiment 3 of the present invention.
Fig. 17B is an enlarged view of a course of a light when the light enters a film 21 illustrated in Fig. 17A with an incident angle $\theta_1$ having a magnitude of a critical angle $\theta c$ in the optical probe according to Embodiment 3 of the present invention.

Description of Embodiments

An absorption coefficient and a scattering coefficient (precisely, a conversion scattering coefficient) among optical characteristic values of a biological tissue are extremely important in a living body diagnosis and the like using a near-infrared light. However, since the light is strongly scattered, it is not easy to measure them in an alive state. Especially, it has been considered impossible to measure absolute values of them with a simple measuring method using a continuous light.

Conventionally, it is premised that a measuring probe has a black contact surface with a living body and a light from an inside of the living body is totally absorbed without reflection by the contact surface. The following describes a technique, conversely, for increasing the reflection of light at a contact surface as much as possible or increasing and decreasing the reflection by changing the reflection in time series or periodically. By applying a theory of light propagation in vivo to such a reflection condition at a contact surface, each of absolute values of the absorption coefficient and the scattering coefficient can be individually determined.

[Embodiment 1]

The following describes an optical characteristic value measuring device according to Embodiment 1 of the present invention in detail with reference to the drawings. In the drawings, the same reference numerals indicate the same or equivalent parts.

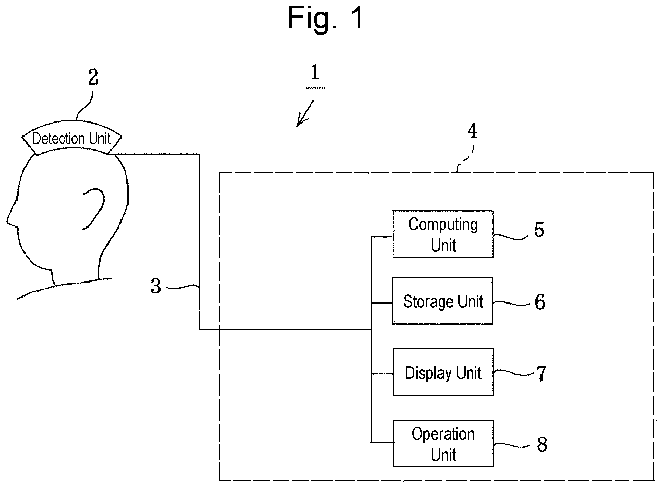

Fig. 1 is a block diagram illustrating an overall configuration of an optical characteristic value measuring device 1 according to Embodiment 1 of the present invention. As illustrated in Fig. 1, the optical characteristic value measuring device 1 includes a detection unit 2 and a calculation unit 4. The detection unit 2 includes a light emission unit that emits a light from a surface to an inside of a head of a subject that is a measurement object, a measurement unit that measures an intensity of the light reflected at the inside and emitted to an outside of the head at at least three points different in distance from the light emission unit on the surface, and a light reflection unit that is placed to cover the surface between the light emission unit and the measurement unit and increases a reflectance of the light emitted from the inside to the outside. The detection unit 2 is placed on the head. The calculation unit 4 is connected to the detection unit 2 via a bus 3, and calculates an optical characteristic value of the measurement object based on the intensity of the light measured by the measurement unit.

The calculation unit 4 includes a computing unit 5, a storage unit 6, a display unit 7, and an operation unit 8 each connected to the bus 3. Here, the computing unit 5 is configured of a Central Processing Unit (CPU), and calculates the absorption coefficient and the scattering coefficient of the measurement object based on the intensity of the light measured by the measurement unit. The calculation method is described later in detail. The storage unit 6 is configured of a semiconductor memory or a hard disk. The storage unit 6 stores programs executed by the computing unit 5, and additionally, the intensity of the light measured by the measurement unit and the values calculated by the computing unit 5. The display unit 7 displays a user interface for operating the device by a user, the above-described calculated values, and the like. The operation unit 8 outputs various kinds of operation instructions to the computing unit 5 corresponding to the operation by the user.

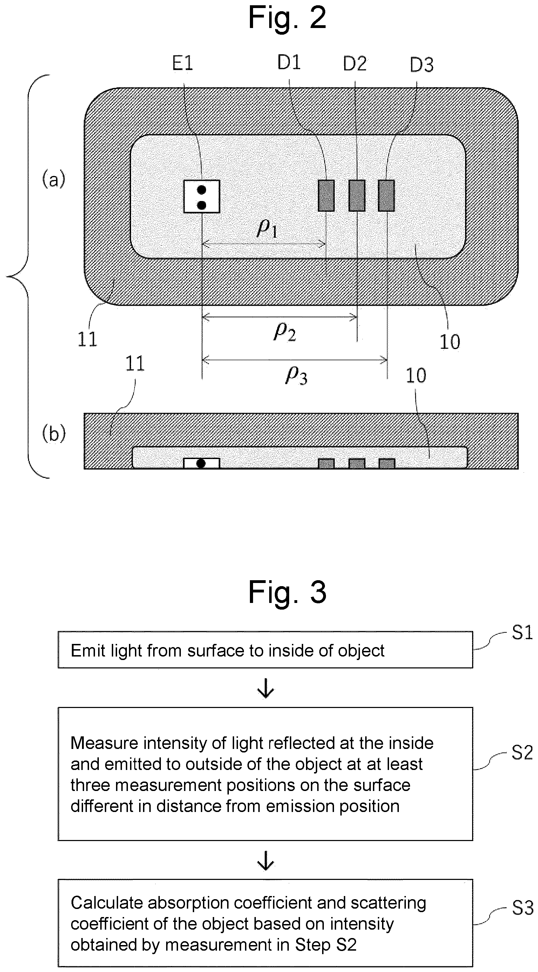

Fig. 2 is a diagram illustrating a structure of an optical probe constituting a detection unit 3 illustrated in Fig. 1. Fig. 2(a) is a bottom view, and Fig. 2(b) is a side view. The bottom surface illustrated in Fig. 2(a) and the lower surface of Fig. 2(b) are brought in contact with the surface of the head.

The optical probe illustrated in Fig. 2 is a minimum unit constituting the detection unit 3, and a plurality of the optical probes constituting the detection unit 3 enables the measurement at a plurality of points of the head.

As illustrated in Fig. 2, the optical probe includes a light emission unit E1, measurement units D1 to D3, a base plate 10, and a cover material 11. The light emission unit E1 is configured of a light-emitting diode, a semiconductor laser, or the like and selectively emits a light having at least two wavelengths from the surface to the inside. The measurement units D1 to D3 are configured of an optical detector, such as an avalanche photodiode and a photodiode. The measurement units D1 to D3 measure the intensity of the light reflected at the inside and emitted to the outside at three points of distances $\rho_1, \rho_2, \rho_3$ from the light emission unit E1 on the surface. The base plate 10 has the bottom surface that is a surface with high reflectance configured of a mirrored surface or a white surface. The base plate 10 is placed to cover the surface between the light emission unit E1 and the measurement unit D1 to D3, thereby increasing the reflectance of the light emitted from the inside to the outside. The cover material 11 is disposed to cover the base plate 10 and configured of a material that absorbs light without reflection.

The base plate 10 is configured of white plastic, white rubber, white powder molding material, white paper, white cloth, white wood, shiny metal foil, or a combination of them, and alternatively, a variable reflectance film.

Fig. 3 is a flowchart illustrating an operation of the optical characteristic value measuring device 1 illustrated in Fig. 1. The following describes the operation of the optical characteristic value measuring device 1 with reference to Fig. 3.

In Step S1, in the optical characteristic value measuring device 1 with the above-described configuration, a light is emitted from the surface to the inside by the light emission unit E1. Next, in Step S2, on the surface, the intensity of the light reflected at the inside and emitted to the outside is measured by the three measurement units D1 to D3. Then, in Step S3, data indicating the intensity of the light obtained by the measurement in Step S2 is stored in the storage unit 6 via the bus 3. The computing unit 5 calculates the absorption coefficient and the scattering coefficient of the head of the subject as the measurement object based on the data stored in the storage unit 6. The following describes the calculation method of the absorption coefficient and the scattering coefficient in detail.

First, the principle of Spatially-resolved Spectroscopy (SRS) by Near-infrared Spectroscopy (NIRS) used in the operation is described in comparison with the principle conventionally used in the same measurement.

Conventionally, continuous light is used in the spatially-resolved spectroscopy that is a technique for estimating a brain activity and a muscle activity by measuring a state of blood in vivo, especially, changes in concentration of oxygenated hemoglobin (HbO2) and deoxygenated hemoglobin (Hb). At this time, when a distance from an emission point to a detection point of a light (hereinafter also referred to as an "SD distance") is $\rho$, and an attenuation of a diffuse reflection light propagating inside a living body and measured at each SD distance from the emission light is A, a relation between an absorption coefficient $\mu_a$ and a scattering coefficient $\mu_s'$ is based on a relation below.

[Formula 1]

$$\mu_a\mu_s' \approx \frac{1}{3}\left(\frac{dA}{d\rho} - \frac{2}{\rho}\right)^2 \approx \frac{1}{3}\left(\frac{A_2 - A_1}{\rho_2 - \rho_1} - \frac{4}{\rho_1 + \rho_2}\right)^2 \qquad (1)$$

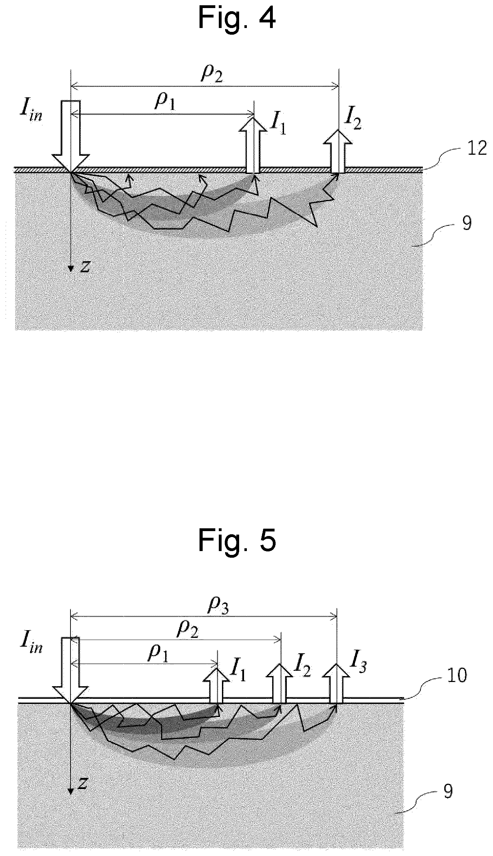

That is, by measuring the attenuation A at a plurality of distances p and obtaining a slope dA/dp, a product $\mu_a\mu_s'$ of the absorption coefficient $\mu_a$ and the scattering coefficient $\mu_s'$ can be determined. At this time, as illustrated in Fig. 4, in the derivation of the formula (1), it is premised that a light entered in a biological tissue 9 with an intensity $I_{in}$ is totally absorbed when reaching a surface 12 and does not return to the inside, that is, a zero boundary condition. Then, there is a problem that the absorption coefficient $\mu_a$ and the conversion scattering coefficient $\mu_s'$ cannot be separately calculated from the formula (1). Note that in such a conventional spatially-resolved spectroscopy, at the distances $\rho_1$, $\rho_2$, the intensities $I_1$, $I_2$ of the diffuse reflection light are measured, respectively. The attenuations $A_1$, $A_2$ are obtained with $A_1 = -\ln(I_1/I_{in})$, $A_2 = -\ln(I_2/I_{in})$, respectively.

In contrast, in the spatially-resolved spectroscopy according to this embodiment, as illustrated in Fig. 5, a light that has reached the surface of the biological tissue 9 is strongly reflected by a surface with high reflectance configured of the base plate 10, and propagated inside the biological tissue 9 again. Then, at measurement points of different distances $\rho_1$, $\rho_2$, $\rho_3$ from the emission point, intensities $I_1$, $I_2$, $I_3$ of the diffuse reflection light are measured, respectively. Diffuse reflectances $R_1$, $R_2$, $R_3$ at the three measurement points are obtained with formulae below, respectively.

[Formula 2]

$$\text{Diffuse Reflectance } R_1 = I_1/I_{in}$$
$$\text{Diffuse Reflectance } R_2 = I_2/I_{in} \qquad (2)$$
$$\text{Diffuse Reflectance } R_3 = I_3/I_{in}$$

Then, by increasing the reflectance (internal reflectance) of the light from the inside of living body at the surface as described above, performing a numerical calculation by a Monte Carlo method simulation for simulating a light propagation phenomenon in vivo and a calculation of an analytical solution of an equation representing the light propagation phenomenon, and matching the calculation result with the measurement result, absolute values of the absorption coefficient $\mu_a$ and the conversion scattering coefficient $\mu_s'$ can be separately calculated. The following describes it in more detail.

Since two variables of the absorption coefficient $\mu_a$ and the conversion scattering coefficient $\mu_s'$ are unknowns, in principle, the absorption coefficient $\mu_a$ and the conversion scattering coefficient $\mu_s'$ can be obtained from the intensities $I_1$, $I_2$ of the diffuse reflection light measured at the two points of the distances $\rho 1$, p2. However, since the measurement of the intensity $I_{in}$ of the emission light is difficult in the actual measurement, it is not easy to obtain the absolute values of the intensities $I_1$, $I_2$ of the diffuse reflection light. Therefore, the intensities $I_1$, $I_2$, $I_3$ of the diffuse reflection light are measured at three points, and simultaneous equations are solved using two of ratios $J_{12}$ (= $I_1/I_2$), $J_{23}$ (= $I_2/I_3$), $J_{13}$ (= $I_1/I_3$) as measurement values, thereby obtaining two unknowns of the absorption coefficient $\mu_a$ and the conversion scattering coefficient $\mu_s'$. Specifically, when the ratios $J_{12}$, $J_{23}$ are used, the simultaneous equations below are used.

[Formula 3]

$$J_{12} = \frac{R(\rho_1;\mu_a,\mu_s')}{R(\rho_2;\mu_a,\mu_s')} \; , \; J_{23} = \frac{R(\rho_2;\mu_a,\mu_s')}{R(\rho_3;\mu_a,\mu_s')} \qquad (3)$$

Here, $R(\rho;\mu_a\mu_s')$ is a diffuse reflectance obtained from a theoretical calculation regarding the light propagation phenomenon using the SD distance p, and the absorption coefficient $\mu_a$ and the conversion scattering coefficient $\mu_s'$ of a medium. The absorption coefficient $\mu_a$ and the conversion scattering coefficient $\mu_s'$ when the ratio of $R(\rho;\mu_a\mu_s')$ on the right side matches J on the left side are values to be obtained.

The formula (3) is transformed, and the absorption coefficient $\mu_a$ and the conversion scattering coefficient $\mu_s'$ are obtained such that two functions $f_1(\mu_a,\mu_s')$, $f_2(\mu_a,\mu_s')$ in a formula (4) below regarding the absorption coefficient $\mu_a$ and the conversion scattering coefficient $\mu_s'$ become zero.

[Formula 4]

$$\left.\begin{array}{l} f_1(\mu_a,\mu_s') = J_{12}R(\rho_2;\mu_a,\mu_s') - R(\rho_1;\mu_a,\mu_s') = 0 \\[2mm] f_2(\mu_a,\mu_s') = J_{23}R(\rho_3;\mu_a,\mu_s') - R(\rho_2;\mu_a,\mu_s') = 0 \end{array}\right\} \qquad (4)$$

The following describes five methods as examples of theoretical calculation of the diffuse reflectance $R(\rho;\mu_a,\mu_s')$.

[1] Method Using Monte Carlo Method Simulation

The Monte Carlo method simulation has been known as a method in which a light is considered as particles (photons) having energy. A state of photons propagating in a medium while being absorbed and scattered is traced point by point. The Monte Carlo method simulation has become practical due to the development of computers. A direction of the photons changed by scattering is provided by a random number so as to statistically indicate a scattering characteristic. A distance traveled before next scattering and an energy loss are provided by random numbers so as to statistically indicate an absorption characteristic.

Here, the method to be used is a look-up table method in which a simulation is performed using various SD distances p, absorption coefficients $\mu_a$, and conversion scattering coefficients $\mu_s'$ to form a table of the diffuse reflectance $R(\rho;\mu_a\mu_s')$ in advance. In this method, a combination of the absorption coefficient $\mu_a$ and the conversion scattering coefficient $\mu_s'$ that satisfies the formula (4) is searched.

[2] Method Using Light Transport Equation

The light transport equation is an equation that expresses an energy conservation law in association with light propagation in a medium in which scattering and absorption occur. It has been known that the light transport equation is expressed by a partial differential and integral equation below.

[Formula 5]

$$\left[\frac{1}{c}\frac{\partial}{\partial t} + \mathbf{s}\nabla + \mu_s + \mu_a\right]I(\mathbf{r},\mathbf{s},t) = \mu_s\int_{4\pi}p(\mathbf{s},\mathbf{s}')I(\mathbf{r},\mathbf{s}',t)d\Omega' + q(\mathbf{r},\mathbf{s},t) \qquad (5)$$

Here, in respective signs, c is a speed of light, $t$ is a time, bold $\mathbf{s}$ is a direction vector, bold $\mathbf{r}$ is a position vector, I is a light intensity, $\mu_s$ is a scattering coefficient, $\mu_a$ is an absorption coefficient, p(bold $\mathbf{s}$, bold $\mathbf{s}'$) is a scattering probability from the direction $\mathbf{s}'$ to the direction $\mathbf{s}$, $d\Omega'$ is an infinitesimal solid angle in the direction $\mathbf{s}'$, and $q$ is a light source intensity inside a medium.

It is also preferable to use the look-up table method by preliminarily performing the numerical calculation under various conditions using the equation indicated as the formula (5) similarly to the case where the Monte Carlo method simulation is used.

[3] Method Using Telegraph Equation

The telegraph equation is an equation in which the directional dependency of light intensity is eliminated through approximation to eliminate the integral term of the light transport equation, thereby converting the light transport equation to a partial differential equation of an integral light intensity $\varphi(r,t)$ including first and second derivatives with respect to time and a second derivative with respect to space as indicated by a formula (6) below. The telegraph equation is known to include a wave nature of light and diffuseness in a scattering medium.

[Formula 6]

$$\left[\frac{D}{c^2}\frac{\partial^2}{\partial t^2}+(1+2D\mu_a)\frac{1}{c}\frac{\partial}{\partial t}+\mu_a(1+D\mu_a)-D\nabla^2\right]\phi(\mathbf{r},t)=q(\mathbf{r},t) \qquad (6)$$

Here, $D = 1/(3\mu_S')$ indicates a light diffusion coefficient.

An analytical solution of the diffuse reflectance obtained by the equation indicated as the formula (6) is obtained in a form of an integral as indicated by a formula (7) below under a boundary condition in which a reflectance (internal reflectance) when a light from an inside of living body reaches a boundary surface (surface) is $r_d$ ($0 < r_d < 1$).

[Formula 7]

$$R(\rho,\mu_a,\mu_s')=\int_0^\infty ds\,\frac{s\left\{\exp\left[-(s-1)(1+\alpha/3)/z_{e0}\right]-\exp\left[-s(1+\alpha)\right]\right\}}{2\pi\left[z_{e0}/(1+\alpha/3)-1/(1+\alpha)\right]}\times\exp\left\{-\left[(s^2+\rho_s^2)\alpha(3+\alpha)\right]^{1/2}\right\}$$

$$\times\frac{1+\left[(s^2+\rho_s^2)\alpha(3+\alpha)\right]^{1/2}}{(s^2+\rho_s^2)^{3/2}} \qquad (7)$$

Here,

[Formula 8]

$$\alpha=\mu_a/\mu_s',\quad z_{e0}=(2/3)(1+r_d)/(1-r_d),\quad \rho_s=\rho\mu_s' \qquad (8)$$

Since an integrand function indicated in the formula (7) rapidly decreases when s increases, a calculation load is not large. Therefore, it is also preferable to use the look-up table method by preliminarily performing the calculation under various conditions.

[4] Method Using Time-Dependent Light Diffusion Equation

A light diffusion equation that is a further approximation of the light transport equation is a partial differential equation of $\varphi(r,t)$ including a first derivative with respect to time and a second derivative with respect to space as indicated by a formula (9) below. However, it is assumed that there is no light source inside the medium.

[Formula 9]

$$\left(\frac{1}{c}\frac{\partial}{\partial t}+\mu_a-D\nabla^2\right)\phi(\mathbf{r},t)=0 \qquad (9)$$

An analytical solution of time-dependent diffuse reflectance in a case of pulse light emission by the equation indicated as the formula (9) is obtained as a formula (10) below under a boundary condition in which the internal reflectance is $r_d$.

[Formula 10]

$$R(\rho,t;\mu_a,\mu_s') = \frac{2Dc}{(4\pi Dct)^{3/2} z_e} \exp\left(-\mu_a ct - \frac{\rho^2 + z_0^2}{4Dct}\right)\left\{1 - \frac{\sqrt{\pi Dct}}{z_e}\exp[h(t)]^2 \operatorname{erfc}[h(t)]\right\}$$

$$(10)$$

[Formula 11]

$$h(t) = \frac{z_0 + 2Dct/z_e}{\sqrt{4Dct}}, \quad \operatorname{erfc}(X) = \frac{2}{\sqrt{\pi}}\int_X^\infty \exp(-t^2)dt \qquad (11)$$

Here,
[Formula 12]

$$D = 1/(3\mu_s'), \quad z_0 = 1/\mu_s', \quad z_e = 2A_R/(3\mu_s'), \quad A_R = (1+r_d)/(1-r_d) \qquad (12)$$

Then, for the diffuse reflectance $R(\rho;\mu_a,\mu_S')$, as indicated by a formula (13) below, it is only necessary to integrate the diffuse reflectance $R(\rho,t;\mu_a,\mu_S')$ with respect to time.
[Formula 13]

$$R(\rho;\mu_a,\mu_s') = \int_0^\infty R(\rho,t;\mu_a,\mu_s')dt \qquad (13)$$

Therefore, it is also preferable to use the look-up table method by preliminarily performing the calculation under various conditions.

[5] Method Using Light Diffusion Equation for Continuous Light

A light diffusion equation for continuous light not depending on time is expressed by a formula (14) below.
[Formula 14]

$$\left(\mu_a - D\nabla^2\right)\phi(\mathbf{r},t) = 0 \qquad (14)$$

An approximate solution of the equation indicated as the formula (14) is easily obtained using an extrapolated boundary method (mirror method). The diffuse reflectance $R(\rho;\mu_a,\mu_S')$ is given by a formula (15) below.
[Formula 15]

$$R(\rho;\mu_a,\mu_s') = \frac{I(\rho;\mu_a,\mu_s')}{I_{in}} = \frac{1}{4\pi}\left[z_0(\mu_e + \frac{1}{r_{10}})\frac{\exp(-\mu_e r_{10})}{r_{10}^2} + (z_0 + 2z_e)(\mu_e + \frac{1}{r_{20}})\frac{\exp(-\mu_e r_{20})}{r_{20}^2}\right]$$

$$(15)$$

Here,
[Formula 16]

$$z_0 = 1/\mu_s', \quad z_e = 2A_R/(3\mu_s')$$

$$r_{10} = (\rho^2 + z_0^2)^{1/2}, \quad r_{20} = [\rho^2 + (z_0 + 2z_e)^2]^{1/2}$$

$$A_R = (1 + r_d)/(1 - r_d), \quad \mu_e = \sqrt{3\mu_a\mu_s'}$$

$$(16)$$

When there is no internal reflection, $r_d = 0$, $A_R = 1$ are met, and the larger the internal reflectance $r_d$ is, the larger $A_R$ becomes. In this case, since the diffuse reflectance $R(\rho;\mu_a,\mu_s')$ is given by the simple formula (15), the simultaneous equations of the formula (4) can be solved analytically. Here, since $z_e$ including $\mu_s'$ becomes large when the internal reflectance $r_d$ is large, $r_{20}$ cannot be approximated to p. Consequently, since the term of $\mu_s'$ appears alone, the absorption coefficient $\mu_a$ and the conversion scattering coefficient $\mu_s'$ can be separately obtained.

In the above description, even when the optical probe illustrated in Fig. 2 employs various structures as described below, the similar operational advantage can be provided.

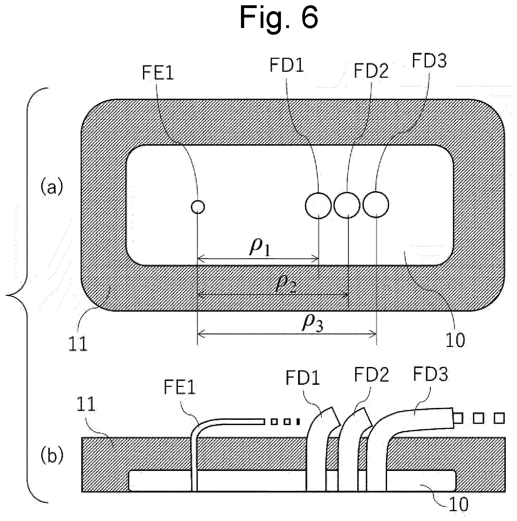

Fig. 6 is a diagram illustrating a second example of the optical probe illustrated in Fig. 2. Fig. 6(a) is a bottom view, and Fig. 6(b) is a side view. As illustrated in Fig. 6, the optical probe according to the second example has a configuration similar to that of the optical probe illustrated in Fig. 2. The optical probe according to the second example is different in that one emission optical fiber FE1 for emitting a light guided from a light source (not illustrated) and three detection optical fibers FD1 to FD3 that are disposed at points spaced from the emission optical fiber FE1 by respective distances $\rho_1, \rho_2, \rho_3$ and guide the detected light to a detector (not illustrated) are provided.

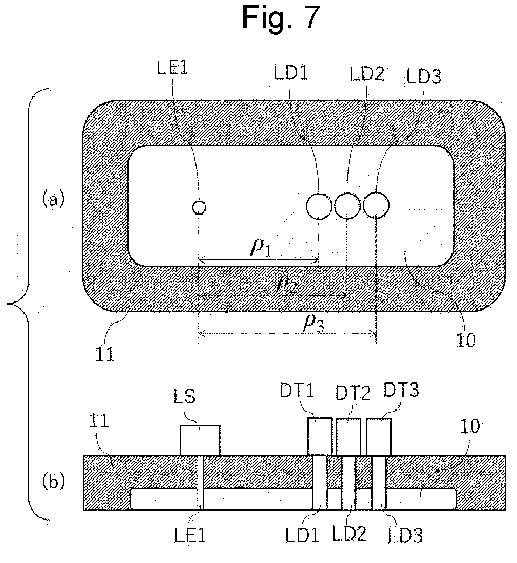

Fig. 7 is a diagram illustrating a third example of the optical probe illustrated in Fig. 2. Fig. 7(a) is a bottom view, and Fig. 7(b) is a side view. As illustrated in Fig. 7, the optical probe according to the third example has a configuration similar to that of the optical probe illustrated in Fig. 2. The optical probe according to the third example is different in that one emission light guide LE1 for emitting a light guided from a light source LS disposed at this optical probe and three detection light guides LD1 to LD3 that are disposed at points spaced from the emission light guide LE1 by respective distances $\rho_1, \rho_2, \rho_3$ and guide the detected light to detectors DT1 to DT3 disposed at this optical probe are provided.

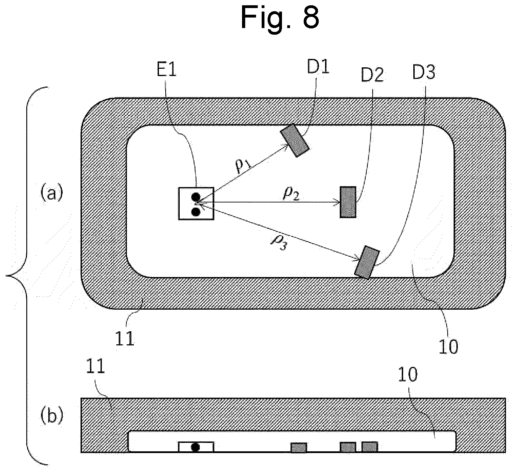

Fig. 8 is a diagram illustrating a fourth example of the optical probe illustrated in Fig. 2. Fig. 8(a) is a bottom view, and Fig. 8(b) is a side view. As illustrated in Fig. 8, the optical probe according to the fourth example has a configuration similar to that of the optical probe illustrated in Fig. 2. The optical probe according to the fourth example is different in that one light emission unit E1 and three measurement units D1 to D3 disposed at positions spaced from the light emission unit E1 by respective distances $\rho_1, \rho_2, \rho_3$ and not in a straight line are provided.

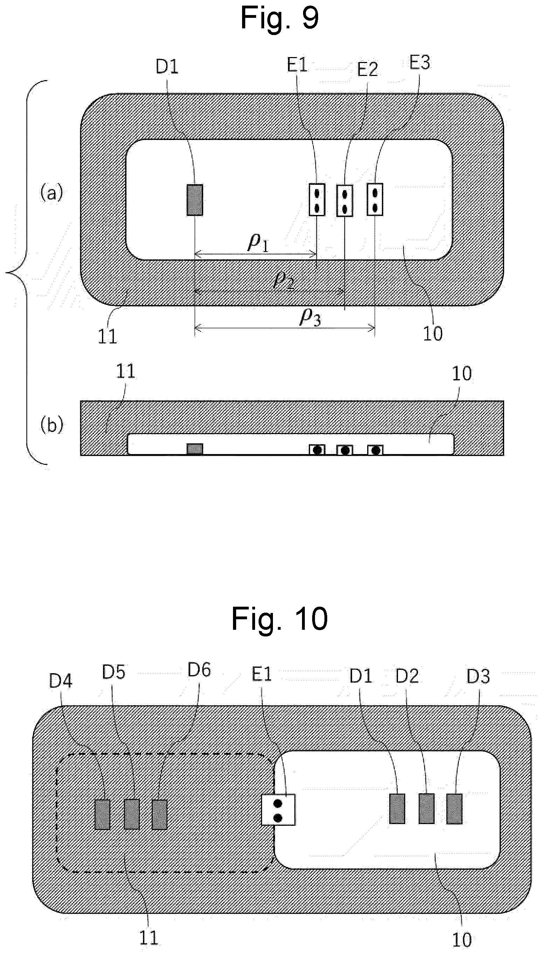

Fig. 9 is a diagram illustrating a fifth example of the optical probe illustrated in Fig. 2. Fig. 9(a) is a bottom view, and Fig. 9(b) is a side view. As illustrated in Fig. 9, the optical probe according to the fifth example has a configuration similar to that of the optical probe illustrated in Fig. 2. The optical probe according to the fifth example is different in that one measurement unit D1 and three light emission units E1 to E3 disposed at positions spaced from the measurement unit D1 by respective distances $\rho_1, \rho_2, \rho_3$ are provided. Note that the three light emission units E1 to E3 do not need to be arranged in a straight line insofar as the three light emission units E1 to E3 are disposed at positions spaced from the measurement unit D1 by the respective distances $\rho_1, \rho_2, \rho_3$.

Fig. 10 is a bottom view illustrating a sixth example of the optical probe illustrated in Fig. 2. As illustrated in Fig. 10, the optical probe according to the sixth example has a configuration similar to that of the optical probe illustrated in Fig. 2. The optical probe according to the sixth example further includes measurement units D4 to D6 that are disposed at three points mutually different in distance from the light emission unit E1 and configured of optical detectors, such as an avalanche photodiode and a photodiode. The base plate 10 with high reflectance is disposed only between the light emission unit E1 and the measurement units D1 to D3.

With this configuration, the intensity can be measured in a state where a reflectance of a light emitted from an inside to an outside of an object is high between the light emission unit E1 and the measurement units D1 to D3 as described above. Further, the intensity can be measured in a state where the reflectance of the light is low between the light emission unit E1 and the measurement units D4 to D6. Thus, the method of calculating the absorption coefficient $\mu_a$ and the conversion scattering coefficient $\mu_s'$ using the intensities measured with the different reflectances is also preferable, and this method is described in Embodiment 2 below.

As described above, with the optical characteristic value measuring device 1 and the method achieved by the operation of the device according to Embodiment 1 of the present invention, the absolute values of the absorption coefficient $\mu_a$ and the conversion scattering coefficient $\mu_s'$ can be calculated. Accordingly, an approximate value of an optical path length of light propagation inside the biological tissue 9 can be calculated by calculating $[(3\mu_s')/(4\mu_a')]^{1/2}\rho$. Therefore, the brain activity and the muscle activity can be estimated with more accuracy from an oxygenation state and the like of blood flowing inside the biological tissue 9.

[Embodiment 2]

The base plate 10 according to Embodiment 1 described above has the constant reflectance. The absolute values of the absorption coefficient $\mu_a$ and the conversion scattering coefficient $\mu_S$' can be calculated even by the similar measurement using a film in which the reflectance is variable instead of the base plate 10. The following describes an embodiment using a variable reflectance film.

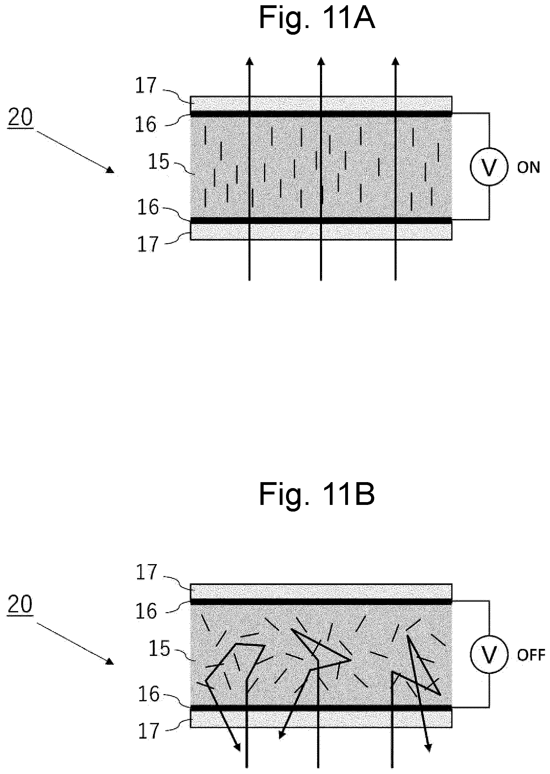

Fig. 11A and Fig. 11B are schematic diagrams for describing a function of a variable reflectance film 20 used in an optical probe constituting a detection unit 2 according to Embodiment 2 of the present invention. Fig. 11A illustrates a state where a voltage is applied. Fig. 11B illustrates a state where a voltage is not applied. As illustrated in Fig. 11A and Fig. 11B, the variable reflectance film 20 includes a liquid crystal layer 15, two electrodes 16 that sandwich the liquid crystal layer 15, and two transparent coating layers 17 disposed outside the respective two electrodes 16.

The use of the variable reflectance film 20 having such a configuration allows actively changing the internal reflectance of the biological tissue 9. That is, since the variable reflectance film 20 is a liquid crystal film having a configuration as described above, as illustrated in Fig. 11A, by applying a voltage in a film thickness direction of the liquid crystal film to align the orientation of liquid crystal molecules in one direction, a light can be transmitted to provide a non-reflective state. Meanwhile, as illustrated in Fig. 11B, by turning the voltage off to make the orientation of the liquid crystal molecules unregulated, the light is diffusely reflected. Therefore, the reflectance in the liquid crystal layer can be variably controlled by changing the voltage. The following describes examples of a method of measuring the absorption coefficient $\mu_a$ and the conversion scattering coefficient $\mu_S$' of the biological tissue 9 by actively changing the internal reflectance.

[1] Case of Measurement with Three Different SD Distances

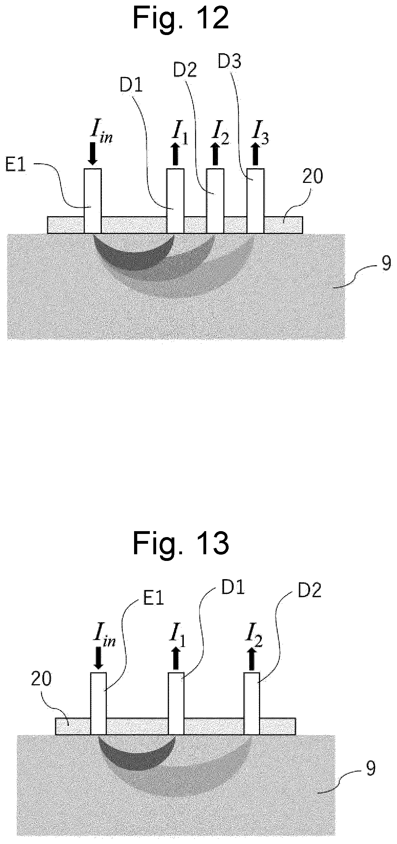

Fig. 12 is a side view illustrating a structure of the optical probe when a measurement is performed with three different SD distances in Embodiment 2 of the present invention. As illustrated in Fig. 12, in this case, using the variable reflectance film 20 having an internal reflectance $r_{d1}$ or an internal reflectance $r_{d2}$, intensities $I_1$, $I_2$, $I_3$ of a diffuse reflection light are measured by three measurement units D1 to D3. Since $J_{12}$ (= $I_1/I_2$) and $J_{23}$ (= $I_2/I_3$) are obtained as measurement values for each of the two internal reflectances $r_{d1}$, $r_{d2}$, simultaneous equations with four unknowns of a formula (17) below are satisfied.

[Formula 17]

$$
\begin{aligned}
J_{12}(r_{d1})R(\rho_2, r_{d1}; \mu_a, \mu_s') - R(\rho_1, r_{d1}; \mu_a, \mu_s') = 0 \\
J_{23}(r_{d1})R(\rho_3, r_{d1}; \mu_a, \mu_s') - R(\rho_2, r_{d1}; \mu_a, \mu_s') = 0 \\
J_{12}(r_{d2})R(\rho_2, r_{d2}; \mu_a, \mu_s') - R(\rho_1, r_{d2}; \mu_a, \mu_s') = 0 \\
J_{23}(r_{d2})R(\rho_3, r_{d2}; \mu_a, \mu_s') - R(\rho_2, r_{d2}; \mu_a, \mu_s') = 0
\end{aligned}
\quad (17)
$$

In the formula (17), the function R is an analytical solution of the diffuse reflectance with the SD distance p, the internal reflectance $r_d$, the absorption coefficient $\mu_a$, and the scattering coefficient $\mu_S$'. Therefore, it is only necessary to solve the simultaneous equations for two unknowns ($\mu_S$', $\mu_a$) or four unknowns ($\mu_S$', $\mu_a$, $r_{d1}$, $r_{d2}$).

[2] Case of Measurement with Two Different SD Distances

Fig. 13 is a side view illustrating a structure of the optical probe when a measurement is performed with two different SD distances in Embodiment 2 of the present invention. As illustrated in Fig. 13, in this case, using the variable reflectance film 20 having an internal reflectance $r_{d1}$ or an internal reflectance $r_{d2}$, intensities $I_1$, $I_2$ of a diffuse reflection light are measured by two measurement units D1, D2.

In this measurement, instead of the ratios $J_{12}$ (= $I_1/I_2$) of the intensities $I_1$, $I_2$ at the different SD distances p for the same internal reflectance $r_d$ are obtained as the measurement values as described above, ratios $J(\rho_1)$ (= $I_1(\rho_1,r_{d1})/I_2(\rho_1,r_{d2})$) and $J(\rho_2)$ (= $I_1(\rho_2,r_{d1})/I_2(\rho_2,r_{d2})$) of the intensities $I_1$, $I_2$ at the same SD distance p are obtained for the different internal reflectances $r_d$. Therefore, simultaneous equations with two unknowns of a formula (18) below are satisfied. Note that simultaneous equations with two unknowns indicated by a formula (19) below using the ratios $J_{12}$ (= $I_1/I_2$) of the intensities $I_1$, $I_2$ at the different SD distances p for the same internal reflectance $r_d$ and the ratios $J(\rho_1)$ (= $I_1(\rho_1,r_{d1})/I_2(\rho_1,r_{d2})$)

of the intensities at the same SD distance p for the different internal reflectances $r_d$ may be used.
[Formula 18]

$$J(\rho_1)R(\rho_1, r_{d2}; \mu_a, \mu_s') - R(\rho_1, r_{d1}; \mu_a, \mu_s') = 0$$
$$J(\rho_2)R(\rho_2, r_{d2}; \mu_a, \mu_s') - R(\rho_2, r_{d1}; \mu_a, \mu_s') = 0 \qquad \bigg\} \qquad (18)$$

[Formula 19]

$$J_{12} R(\rho_2, r_{d1}; \mu_a, \mu_s') - R(\rho_1, r_{d1}; \mu_a, \mu_s') = 0$$
$$J(\rho_1)R(\rho_1, r_{d2}; \mu_a, \mu_s') - R(\rho_1, r_{d1}; \mu_a, \mu_s') = 0 \qquad \bigg\} \qquad (19)$$

It is only necessary to solve the simultaneous equations indicated as the formula (18) or the formula (19) for two unknowns ($\mu_s'$, $\mu_a$). This is preferable in that the number of required measurement units and the number of required measurements are small compared with the case where the measurement is performed with three different SD distances, and the solution is easily obtained.

[3] Case of Modulating Internal Reflectance of Variable Reflectance Film 20 at Frequency $\omega$

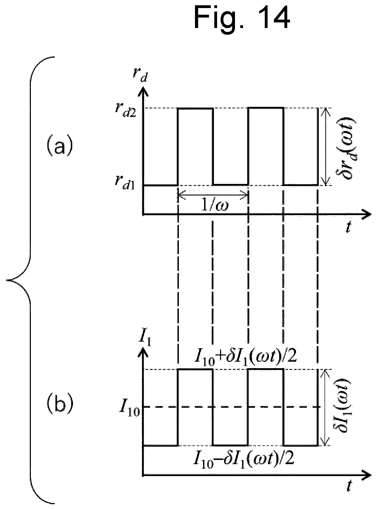

Fig. 14 is a graph for describing a modulation method of an internal reflectance of the variable reflectance film 20 illustrated in Fig. 13. Fig. 14(a) illustrates a temporal change of an internal reflectance $r_d$ of the variable reflectance film 20. Fig. 14(b) illustrates a temporal change of an intensity $I_1$ of a detected light measured by a measurement unit D1 illustrated in Fig. 13.
As illustrated in Fig. 14(a), the internal reflectance of the variable reflectance film 20 is modulated at the frequency $\omega$. An intensity of a modulated component of a diffuse reflection light is measured by a lock-in amplifier method using the frequency $\omega$. The intensity $I_1$ at this time and measurement values J(p) by the measurement units D1, D2 illustrated in Fig. 13 are measured as follows.
The intensity $I_1$ detected at the SD distance $\rho_1$ can be expressed as $[I_{10}+\delta I_1(\omega t)]$ using a steady-state value $I_{10}$ of the intensity after a low-pass filter. Therefore, a formula (20) below is satisfied.
[Formula 20]

$$J(\rho_1) = \{I_{10} - |\delta I_1(\omega t)|/2\}/\{I_{10} + |\delta I_1(\omega t)|/2\} \qquad (20)$$

In the formula (20), an absolute value of $\delta I_1(\omega t)$ indicates an output value of the lock-in amplifier at the frequency $\omega$.
Incidentally, a control voltage and the internal reflectance of the variable reflectance film 20 have a functional relation. Therefore, a waveform of the control voltage to the variable reflectance film 20 can be formed such that a periodic average of a variable term $\delta I_1(\omega t)$ becomes zero. Accordingly, the formula (20) can be easily satisfied.
For the intensity $I_2$, a formula (21) below is similarly satisfied.
[Formula 21]

$$J(\rho_2) = \{I_{20} - |\delta I_2(\omega t)|/2\}/\{I_{20} + |\delta I_2(\omega t)|/2\} \qquad (21)$$

The values $J(\rho_1)$, $J(\rho_2)$ obtained by the formula (20) and the formula (21) are preferable because of being highly resistant to noise and the like and having a high measurement accuracy compared with the case where the measurement is performed for only a direct current component at the two different SD distances. In this case, simultaneous equations are as follows.
[Formula 22]

$$R(\rho_1, r_{d2}; \mu_a, \mu_s')\{I_{10} - |\delta I_1(\omega t)|/2\}/\{I_{10} + |\delta I_1(\omega t)|/2\} - R(\rho_1, r_{d1}; \mu_a, \mu_s') = 0$$
$$R(\rho_2, r_{d2}; \mu_a, \mu_s')\{I_{20} - |\delta I_2(\omega t)|/2\}/\{I_{20} + |\delta I_2(\omega t)|/2\} - R(\rho_2, r_{d1}; \mu_a, \mu_s') = 0 \qquad \bigg\} \qquad (22)$$

[Embodiment 3]

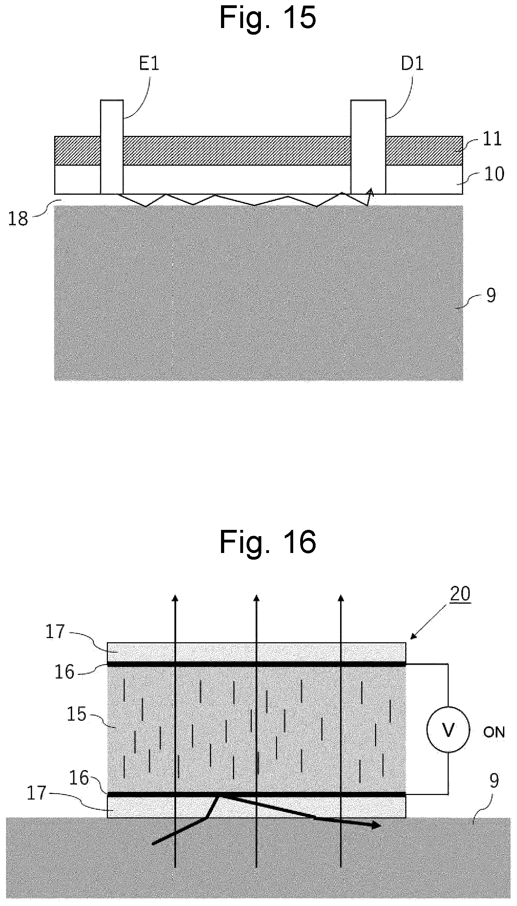

In the optical probe as described above, when a contact surface with a biological surface is not a non-reflective surface or a total absorption surface but a surface with high reflectance, the contact state is insufficient, and a gap may be formed between the optical probe and the biological surface. Then, as illustrated in Fig. 15, a phenomenon referred to as a light piping or a light channeling in which a light propagates along a gap 18 occurs, and a light not propagating inside the biological tissue 9 is possibly detected. In the case of the non-reflective surface, even when a gap is slightly formed, it is not a problem because a light is absorbed by an optical probe surface.

When the variable reflectance film 20 as described above is used, it is concerned that the transparent coating layer 17 is in contact with the biological tissue 9 in a liquid crystal film having a structure as illustrated in Fig. 16. A light piping as indicated by arrows in Fig. 16 occurs transmitting through the transparent coating layer 17. In such a light piping, a light is reflected at an interface between the transparent coat layer 17 and the liquid crystal layer 15. Thereby, a problem arises in that a contrast between on and off of a voltage applied to the variable reflectance film 20 is reduced.

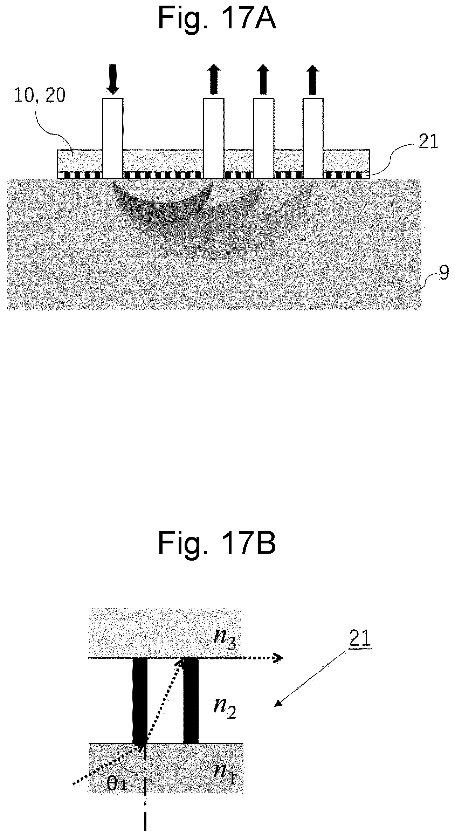

Therefore, to avoid such a problem, it is preferable to insert a film 21 having a louver structure with a blocking effect to an oblique incident light as illustrated in Fig. 17A between the base plate 10 or the variable reflectance film 20 and the biological tissue 9.

As illustrated in Fig. 17B, when refractive indexes of the biological tissue 9, the film 21, and the transparent coating layer 17 of the variable reflectance film 20 or the base plate 10 are $n_1$, $n_2$, $n_3$, respectively, a magnitude relation thereof is assumed as $n_1 < n_2, n_3$. At this time, when an incident angle of a light entering the film 21 from the biological tissue 9 is $\theta_1$, the light is transmitted through the film 21 and enters the base plate 10 or the variable reflectance film 20 in a case where the incident angle $\theta_1$ is smaller than a critical angle $\theta_c$ ($= \sin^{-1}(n_1/n_3)$). The light is totally reflected by the transparent coating surface 17 in a case where the incident angle $\theta_1$ is equal to or more than the critical angle $\theta_c$. This allows avoiding the light piping.

Reference Signs List

    1 Optical characteristic value measuring device
    2 Detection unit
    5 Computing unit
    10 Base plate
    20 Variable reflectance film
    21 Film
    E1 to E3 Light emission unit
    D1 to D6 Measurement unit
    FE1 Emission optical fiber
    FD1 to FD3 Detection optical fiber
    LE1 Emission light guide
    LD1 to LD3 Detection light guide
    LS Light source
    DT1 to DT3 Detector

**Claims**

1. An optical characteristic value measuring device comprising:

    a light emission means configured to emit a light from a surface to an inside of an object;
    a measurement means configured to measure an intensity of the light reflected at the inside and emitted to an outside of the object at at least two points on the surface mutually different in distance from the light emission means;
    a light reflection means that is placed to cover the surface between the light emission means and the measurement means and is configured to increase a reflectance of the light emitted from the inside to the outside; and
    a computing means configured to calculate an absorption coefficient and a scattering coefficient of the object based on the intensity obtained by the measurement.

2. The optical characteristic value measuring device according to claim 1,

wherein the measurement means is configured to measure the intensity of the light reflected at the inside and emitted to the outside of the object at three points on the surface mutually different in distance from the light emission means.

3. The optical characteristic value measuring device according to claim 1,
   wherein an internal reflectance of the light reflection means is variable.

4. The optical characteristic value measuring device according to claim 3,
   wherein the internal reflectance is periodically modulated.

5. The optical characteristic value measuring device according to claim 1, further comprising
   a light blocking means disposed between the object and the light reflection means to avoid a light piping that occurs at a boundary between the object and the light reflection means.

6. The optical characteristic value measuring device according to claim 1,
   wherein the computing means is configured to calculate a diffuse reflectance at the object of the light by a Monte Carlo method simulation to calculate the absorption coefficient and the scattering coefficient.

7. The optical characteristic value measuring device according to claim 1,
   wherein the computing means is configured to calculate a diffuse reflectance at the object of the light as a solution of a telegraph equation to calculate the absorption coefficient and the scattering coefficient.

8. The optical characteristic value measuring device according to claim 1,
   wherein the computing means is configured to calculate a diffuse reflectance at the object of the light as a solution of a light diffusion equation to calculate the absorption coefficient and the scattering coefficient.

9. The optical characteristic value measuring device according to claim 1,
   wherein the computing means is configured to calculate a diffuse reflectance at the object of the light as a solution of a light transport equation to calculate the absorption coefficient and the scattering coefficient.

10. An optical characteristic value measuring device, comprising:

    a measurement means that is disposed on a surface of an object and is configured to measure an intensity of a light emitted from an inside to an outside of the object;
    a light emission means configured to selectively emit the light from the surface to the inside at at least three points on the surface mutually different in distance from the measurement means;
    a light reflection means that is placed to cover the surface between the light emission means and the measurement means and is configured to increase a reflectance of the light emitted from the inside to the outside; and
    a computing means configured to calculate an absorption coefficient and a scattering coefficient of the object based on the intensity obtained by the measurement.

11. An optical characteristic value measuring method in an optical characteristic value measuring device in which a light reflection means is placed between an emission position of a light from a surface to an inside of an object and a measurement position at which an intensity of the light reflected at the inside and emitted to an outside of the object is measured and the light reflection means is configured to increase a reflectance of the light emitted from the inside to the outside covers the surface, the method comprising:

    a first step of emitting the light from the surface to the inside;
    a second step of measuring the intensity of the light reflected at the inside and emitted to the outside of the object at at least two points on the surface mutually different in distance from the emission position; and
    a third step of calculating an absorption coefficient and a scattering coefficient of the object based on the intensity obtained by the measurement in the second step.

12. The optical characteristic value measuring method according to claim 11,
    wherein in the third step, a diffuse reflectance at the object of the light is calculated by a Monte Carlo method simulation to calculate the absorption coefficient and the scattering coefficient.

13. The optical characteristic value measuring method according to claim 11,
    wherein in the third step, a diffuse reflectance at the object of the light is calculated as a solution of a telegraph equation to calculate the absorption coefficient and the scattering coefficient.

14. The optical characteristic value measuring method according to claim 11,
    wherein in the third step, a diffuse reflectance at the object of the light is calculated as a solution of a light diffusion
    equation to calculate the absorption coefficient and the scattering coefficient.

15. The optical characteristic value measuring method according to claim 11,
    wherein in the third step, a diffuse reflectance at the object of the light is calculated as a solution of a light transport
    equation to calculate the absorption coefficient and the scattering coefficient.

# Fig. 1

Detection Unit

Computing Unit — 5

Storage Unit — 6

Display Unit — 7

Operation Unit — 8

# Fig. 2

E1     D1    D2    D3

(a)

11    11

$\rho_1$

$\rho_2$

$\rho_3$

10    10

(b)

# Fig. 3

| | |
|---|---|
| Emit light from surface to inside of object | S1 |

↓

| | |
|---|---|
| Measure intensity of light reflected at the inside and emitted to outside of the object at at least three measurement positions on the surface different in distance from emission position | S2 |

↓

| | |
|---|---|
| Calculate absorption coefficient and scattering coefficient of the object based on intensity obtained by measurement in Step S2 | S3 |

# Fig. 4

# Fig. 5

# Fig. 6

# Fig. 7

(a)

LE1    LD1    LD2    LD3

$\rho_1$

$\rho_2$

$\rho_3$

11    10

(b)

11    LS    DT1   DT2   DT3    10

LE1    LD1   LD2   LD3

# Fig. 8

# Fig. 9

# Fig. 10

## Fig. 11A

## Fig. 11B

# Fig. 12

# Fig. 13

# Fig. 14

## Fig. 15

## Fig. 16

# Fig. 17A

10, 20

21

9

# Fig. 17B

$n_3$

21

$n_2$

$n_1$

$\theta_1$

## INTERNATIONAL SEARCH REPORT

International application No.

**PCT/JP2023/015877**

### A. CLASSIFICATION OF SUBJECT MATTER

***G01N 21/17***(2006.01)i; ***G01N 21/49***(2006.01)i
FI:    G01N21/17 610; G01N21/49 A

According to International Patent Classification (IPC) or to both national classification and IPC

### B. FIELDS SEARCHED

Minimum documentation searched (classification system followed by classification symbols)

G01N21/00-G01N21/61; G01N33/00-G01N33/98; A61B5/145-A61B5/1495

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); Scopus

### C. DOCUMENTS CONSIDERED TO BE RELEVANT

| Category* | Citation of document, with indication, where appropriate, of the relevant passages | Relevant to claim No. |
|---|---|---|
| X | JP 9-178661 A (BOEHRINGER MANNHEIM GMBH) 11 July 1997 (1997-07-11) paragraphs [0006]-[0011], [0033], [0043]-[0084], fig. 1-7 | 1, 3, 10-11 |
| Y | | 2, 6-9, 12-15 |
| A | | 4-5 |
| Y | JP 10-500338 A (BOEHRINGER MANNHEIM GMBH) 13 January 1998 (1998-01-13) p. 25, line 19 to p. 30, line 18, fig. 5-11 | 2 |
| Y | US 2004/152089 A1 (KRAEMER, Uwe) 05 August 2004 (2004-08-05) paragraphs [0036]-[0060], fig. 1-7 | 2 |
| Y | JP 8-136448 A (HAMAMATSU PHOTONICS KK) 31 May 1996 (1996-05-31) paragraphs [0016]-[0019] | 6, 12 |

☑ Further documents are listed in the continuation of Box C.    ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **16 June 2023** | **04 July 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

International application No.

**PCT/JP2023/015877**

### C. DOCUMENTS CONSIDERED TO BE RELEVANT

| Category* | Citation of document, with indication, where appropriate, of the relevant passages | Relevant to claim No. |
|---|---|---|
| Y | 清水 孝一, 生体組織における光伝搬の解析, 光学, August 2012, vol. 41, no. 8, 414(2)-423(11), https://annex.jsap.or.jp/photonics/kogaku/public/41-08-kaisetsu1.pdf<br>in particular, 1. Analysis of light propagation, 1.1 Radiative transfer equation, 1.2 Diffusion equation, 2. Analysis by diffusion equation, 2.3 Analysis of diffusion phenomena, (SHIMIZU, Koichi. Analysis of Light Propagation in Biological Tissue. Kogaku.) | 7-9, 13-15 |
| Y | 東森 信就, 輸送方程式の拡散方程式近似および電信方程式近似に対する逆問題の相違について, 第62回理論応用力学講演会 講演論文集, セッションID: OS05-07, 26 March 2013, https://doi.org/10.11345/japannctam.62.0.253.0<br>in particular, 1. Introduction, 3. Diffusion Approximation, 4. the telegraph equation approximation, (HIGASHIMORI, Nobuyuki. Differences in inverse problems posed for the diffusion equation approximation and the telegraph equation approximation of the transport equation.), non-official translation (Lecture Proceedings of The 62nd Japan National Congress for Theoretical and Applied Mechanics. session ID: OS05-07.) | 7-9, 13-15 |
| A | JP 8-285764 A (AGENCY OF IND SCIENCE & TECHNOL) 01 November 1996 (1996-11-01)<br>entire text, all drawings | 1-15 |
| A | US 2007/0201788 A1 (LIU, Quan) 30 August 2007 (2007-08-30)<br>entire text, all drawings | 1-15 |

EP 4 517 298 A1

INTERNATIONAL SEARCH REPORT
Information on patent family members

International application No.

PCT/JP2023/015877

| Patent document cited in search report | | | Publication date (day/month/year) | Patent family member(s) | | | Publication date (day/month/year) |
|---|---|---|---|---|---|---|---|
| JP | 9-178661 | A | 11 July 1997 | US | 5825488 | A | |
| | | | | column 2, line 1 to column 4, line 7, column 5, line 1 to column 9, line 26, fig. 1-7 | | | |
| | | | | EP | 774658 | A2 | |
| | | | | DE | 19543020 | A1 | |
| JP | 10-500338 | A | 13 January 1998 | US | 5770454 | A | |
| | | | | column 11, line 52 to column 14, line 50, fig. 5-11 | | | |
| | | | | WO | 1995/032416 | A1 | |
| | | | | EP | 760091 | A1 | |
| | | | | AU | 2342595 | A | |
| US | 2004/0152089 | A1 | 05 August 2004 | WO | 2002/069790 | A2 | |
| | | | | p. 12, line 11 to p. 18, line 27, fig. 1-7 | | | |
| | | | | EP | 1365681 | A2 | |
| | | | | DE | 10110599 | A1 | |
| | | | | AU | 2002251002 | A1 | |
| JP | 8-136448 | A | 31 May 1996 | US | 5676142 | A | |
| | | | | column 5, lines 25-65 | | | |
| | | | | EP | 710832 | A1 | |
| JP | 8-285764 | A | 01 November 1996 | (Family: none) | | | |
| US | 2007/0201788 | A1 | 30 August 2007 | WO | 2007/100648 | A2 | |
| | | | | entire text, all drawings | | | |
| | | | | EP | 1988829 | A2 | |

Form PCT/ISA/210 (patent family annex) (January 2015)

29

**EP 4 517 298 A1**

**Patent documents cited in the description**

- JP H0749304 A